# FASCICULE DE BREVET EUROPEEN

(11) **EP 4 166 114 B1**
(45) Date de publication et mention de la délivrance du brevet: **17.04.2024**
(21) Numéro de dépôt: 22199641.6
(22) Date de dépôt: 04.10.2022
(51) Int. Cl.: A61F 2/32, A61F 2/38, A61F 2/40

(54) **IMPLANT AVEC SURFACE ARTICULAIRE EN CÉRAMIQUE**
IMPLANTAT MIT KERAMISCHER GELENKFLÄCHE
IMPLANT WITH CERAMIC ARTICULAR SURFACE

(30) Priorité: 15.10.2021 FR 2110993; 29.10.2021 FR 2111538
(43) Date de publication de la demande: 19.04.2023
(73) Titulaire: FX Shoulder Solutions, 01440 Viria (FR)
(72) Inventeur: DAUDET, Jean-Marie, 01000 BOURG-EN-BRESSE (FR)
(74) Mandataire: LLR

(56) Documents cités:
- EP-A1- 1 952 788
- US-A1- 2014 025 173

## Description

L'invention concerne un implant de prothèse articulaire. Plus spécifiquement, l'invention concerne un implant de prothèse articulaire dont la surface externe articulaire est en céramique.

On connaît déjà dans l'état la technique, des prothèses articulaires de plusieurs types, par exemple des prothèses d'épaule, des prothèses de hanche ou encore des prothèses de genou.

L'ensemble de ces prothèses articulaires comprennent deux implants présentant chacun une surface externe articulaire destinée à venir en contact l'une avec l'autre, reproduisant ainsi l'articulation. Afin de reproduire la meilleure qualité d'articulation possible, il est connu d'utiliser pour ces surfaces externes articulaires en contact, des couples de matériaux favorables, ces couples pouvant être deux matériaux dits « dur » ou un matériau « dur » et un matériau dit « mou ». Les matériaux « durs » connus et utilisés dans les implants de prothèse articulaires sont l'alliage de Cobalt Chrome, alliage de titane acier inox ou encore la céramique. Les matériaux dits « mous » peuvent être en plastique, par exemple en polyéthylène de masse molaire très élevée (UHMWPE) ou en polyétheréthercétone (PEEK) ou en PEKK(poly ether ketone ketone) et leurs dérivés.

Le Cobalt Chrome est un alliage particulièrement apprécié des fabricants d'implants de prothèse articulaire car il présente des propriétés tribologiques et mécaniques exceptionnelles qui permettait notamment de visser et serrer l'implant concerné (à l'aide d'une ou plusieurs vis localisées dans l'implant) soit directement dans l'os du patient, soit dans un élément lui-même fixé à l'os du patient (par exemple une métaglène pour une prothèse totale d'épaule inversée), et ce, sans risque de détérioration de l'implant.

Cependant, les dernières normes en vigueur (et notamment la norme mise à jour du règlement délégué (UE) 2020/217 du 4 octobre 2019 REACH) tendent à interdire l'utilisation du chrome cobalt comme matériau pour les implants de prothèse articulaire au motif que le cobalt est considéré comme une substance potentiellement cancérigène, mutagène ou toxique pour le patient. Dès lors, il convient d'envisager des matériaux durs alternatifs.

Un matériau de type dur connu est la céramique. Ce matériau présente l'avantage d'être biocompatible et particulièrement adapté pour les frottements au regard de ses propriétés de dureté. Cependant, la céramique est un matériau très peu ductile et les contraintes mécaniques très fortes et localisées résultantes du serrage précité au moyen d'une ou plusieurs vis risquent d'engendrer des fissures au sein de la céramique, voire même une rupture de cette dernière. Un tel risque n'est pas acceptable que ce soit pour le chirurgien lors de l'opération ou pour le patient qui risque de devoir remplacer sa prothèse trop rapidement. De plus, il n'est actuellement pas autorisé de remettre un implant en céramique après une rupture de la céramique d'un premier implant. Il est dès lors obligatoire de s'orienter vers un autre couple de matériaux.

Document US2014/025173 décrit un implant selon le préambule de la revendication 1.

L'invention a notamment pour but de permettre l'utilisation d'un tel implant en céramique qui surmonte l'ensemble des inconvénients précités.

A cet effet l'invention a pour objet un implant de prothèse articulaire comprenant :
- un corps principal principalement en céramique et comprenant une surface externe articulaire en céramique de forme concave ou convexe,
- au moins une vis de serrage configurée pour permettre le serrage du corps principal sur l'os du patient ou sur un élément intermédiaire fixé audit os,
- au moins une bague rapportée dans le corps principal et formant avec ce dernier une chambre emprisonnant la tête de la vis de serrage et comprenant une surface d'appui formant butée pour ladite tête de la vis de serrage, la bague rapportée étant configurée pour être en contact avec la vis de serrage durant ledit serrage, la bague rapportée comprenant un orifice central traversée par la vis de serrage, la bague rapportée étant configurée pour répartir les contraintes mécaniques issues du serrage de la vis de serrage de manière homogène au sein du corps principal.

Ainsi, il est possible de réaliser un implant pour prothèse articulaire en céramique (ou principalement en céramique) qui soit à la fonctionnel et robuste et qui ne risque pas de fissurer ou de casser lors du serrage de la vis de serrage. En effet, la présence de la bague rapportée présente le double avantage de participer à l'emprisonnement de la tête de vis, limitant ainsi celle-ci dans ses mouvements, et réalise une surface d'appui contre laquelle vient buter la tête de la vis de serrage lors du serrage. Le contact entre la bague rapportée et la vis de serrage permet de répartir les contraintes mécaniques issues du serrage de la vis de manière homogène au sein du corps principal en céramique, évitant ainsi que ces efforts ne se répercutent que sur des zones spécifiques ce qui entrainerait l'apparition de fissures ou une cassure du corps principal.

La bague rapportée peut être réalisée en tout matériaux permettant une bonne répartition des contraintes mécaniques au sein du corps principal. La bague rapportée peut notamment être réalisée tout ou partie en métal, en plastique ou en céramique. Elle peut également être réalisée avec plusieurs matériaux différents dont une composition des matériaux précités.

Par réparation homogène des contraintes mécaniques issues du serrage de la vis, on entend que les contraintes sont réparties sur toute la surface du corps principal en contact avec la bague rapporté ce qui permet ainsi d'éviter que ne s'applique sur le corps principal en céramique une contrainte qui serait supérieure à la limite élastique et qui entrainerait une rupture de la céramique. Grâce à une grande surface de contact radiale de la bague rapportée avec le corps principal, il est possible d'avoir une répartition des forces sur une surface plus importante du corps principal pour que les contraintes localisées soient les plus faibles possibles.

Suivant d'autres caractéristiques optionnelles de l'invention prises seules ou en combinaison :
- la bague rapportée comprend une portion filetée le corps principal comprend une portion taraudée correspondante, la portion filetée et la portion taraudée étant configurées pour permettre le vissage de la bague rapportée avec le corps principal ;
- la bague rapportée comprend des moyens de clipsage au corps principal ;
- la bague rapportée comprend des moyens périphériques déformables élastiquement configurés pour se déformer au passage de ladite vis de serrage ;
- la bague rapportée est fendue de sorte à pouvoir s'élargir au passage de ladite vis de serrage ;
- la bague rapportée est réalisée dans un matériau thermiquement expansif exerçant une force de pression d'expansion sur le corps principal ;
- la bague rapportée est rapportée brasée avec le corps principal ;
- la bague rapportée est collée sur le corps principal ;
- le corps principal est une glénosphère, la vis de serrage étant configurée pour permettre le serrage entre la glénosphère et une métaglène fixée sur la glène du patient ;
- la glénosphère a un diamètre compris entre 30 et 46mm ;
le corps principal est un plateau tibial céramique, un condyle de genou, une glène anatomique, un cotyle de hanche ou une cupule céramique.

L'invention a également pour objet une prothèse articulaire comprenant un implant selon l'une quelconque des variantes de l'invention précédentes.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue éclatée (figure 1A) et en coupe (figures 1B et 1C) d'une première variante du premier mode de réalisation où la bague est vissée au sein du logement, situé dans le dôme de la glénosphère en céramique,
Fig. 2] la figure 2 est une vue éclatée (figure 2A) et en coupe (figures 2B et 2C) d'une deuxième variante du premier mode de réalisation où la bague est clipsée dans le logement, situé dans le dôme de la glénosphère en céramique,
[Fig. 3] la figure une vue éclatée (figure 3A) et en coupe (figures 3B et 3C) d'une troisième variante du premier mode de réalisation où la bague est également clipsée dans le logement, situé dans le dôme de la glénosphère en céramique,
[Fig. 4] la figure 4 est une vue une vue éclatée (figure 4A) et en coupe (figures 4B et 4C) d'une quatrième variante du premier mode de réalisation où la bague est rapportée frettée dans le logement, situé dans le dôme de la glénosphère en céramique,
[Fig. 5] la figure 5 est une vue une vue éclatée (figure 5A) et en coupe (figures 5B et 5C) d'une cinquième variante du premier mode de réalisation où la bague est rapportée brasée dans le logement, situé dans le dôme de la glénosphère en céramique,
[Fig. 6] la figure 6 est deux vues en coupe illustrant une position libre (figure 6A) et une position de serrage de l'implant (figure 6B) selon la deuxième variante du premier mode de réalisation,
[Fig. 7] la figure 7 est deux vues en coupe d'une première variante du deuxième mode de réalisation comprenant deux bagues (dont une seule est illustrée), chacune clipsée au sein d'un logement situé dans la glène anatomique, illustrant une position libre (figure 7A) et une position de serrage de l'implant (figure 7B).

### Description détaillée

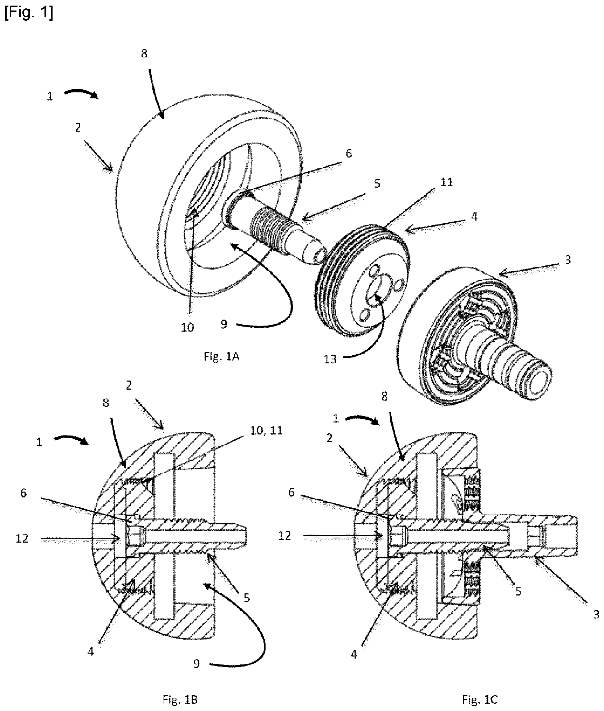

On a représenté sur les figures 1A à 6B un implant de prothèse articulaire selon un premier mode de réalisation de l'invention, lui-même décliné selon plusieurs variantes, l'implant selon ce premier mode de réalisation étant désigné par la référence générale 1.

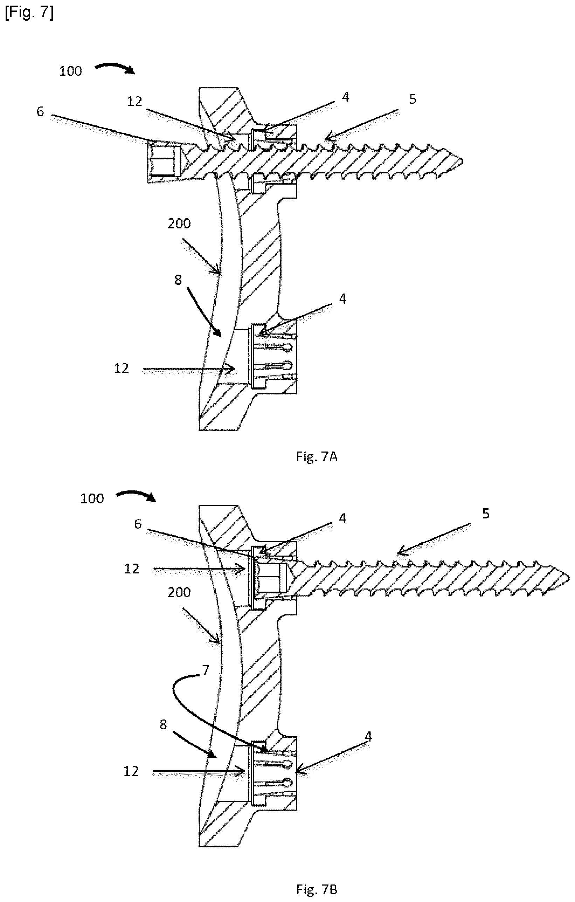

On a représenté sur les figures 7A et 7B un implant de prothèse articulaire selon un deuxième mode de réalisation de l'invention, l'implant selon ce premier mode de réalisation étant désigné par la référence générale 100.

Pour ces deux modes de réalisation illustrés, ainsi que pour ceux non illustrés, l'implant de prothèse articulaire 1 ou 100 comprend :
- un corps principal 2 ou 200 principalement en céramique et comprenant une surface externe articulaire 8 en céramique de forme concave ou convexe,
- au moins une vis de serrage 5 configurée pour permettre le serrage du corps principal 2 ou 200 sur l'os du patient (non représenté) ou sur un élément intermédiaire 3 fixé audit os,
- au moins une bague rapportée 4 dans le corps principal 2 ou 200 et formant avec ce dernier une chambre 12 emprisonnant la tête 6 de la vis de serrage 5 et comprenant une surface d'appui formant butée pour ladite tête de vis, la bague étant configurée pour être en contact avec la vis 5 durant ledit serrage, la bague 4 comprenant un orifice central 13 traversée par la vis 5, la bague 4 étant configurée pour répartir les contraintes mécaniques issues du serrage de la vis 5 de manière homogène au sein du corps principal 2 ou 200.

Il convient de noter que les différentes variantes illustrées pour le premier mode de réalisation, dans lequel l'implant est un implant glénoïdien de prothèse d'épaule inversée, peuvent s'appliquer à toute prothèse comprenant un implant articulaire présentant une surface articulaire en céramique et étant vissé et serré au moyen d'au moins une vis de serrage, sur l'os du patient ou sur un élément intermédiaire fixé audit os, comme cela est notamment le cas du deuxième mode de réalisation illustré, dans lequel l'implant est un implant glénoïdien de prothèse d'épaule anatomique comprenant une bague rapportée 4 correspondant à celle de la deuxième variante du premier mode de réalisation.

Le type de céramique utilisé pour le corps principal peut être tout type de céramique connu de l'homme du métier. A titre d'exemple, il peut s'agir de l'alumine, de la zircone ou d'un matériau composite. La céramique étant un matériau de type dur, il peut être utilisé dans le cadre de couple de matériaux « dur/dur » ou « dur-mou ». Dans ce second exemple, le matériau dit « mou » peut être du polyéthylène de masse molaire très élevée (UHMWPE), du polyétheréthercétone (PEEK), du PEKK(poly ether ketone ketone) ou leurs dérivés

Dans le premier mode de réalisation (figures 1A à 6B), le corps principal 2 correspond à une glénosphère 2 dont la vis de serrage 5 permet le serrage avec un élément intermédiaire 3 fixé à l'os, en l'occurrence la métaglène 3.

La glénosphère 2 en céramique peut avoir un diamètre allant de 30 à 46mm, peut être centrée ou excentrée et peut être latéralisée le cas échéant.

La glénosphère 2 et la métaglène 3 sont assemblées l'une à l'autre par l'intermédiaire de la vis de serrage 5. Afin d'assurer un assemblage encore meilleur, un cône morse (non représenté) est prévu entre la glénosphère 2 et la métaglène 3.

La glénosphère 2 comprend un logement 9 dimensionné pour recevoir la bague rapportée 4 et formé avec cette dernière la chambre 12 emprisonnant la tête 6 de la vis de serrage 5.

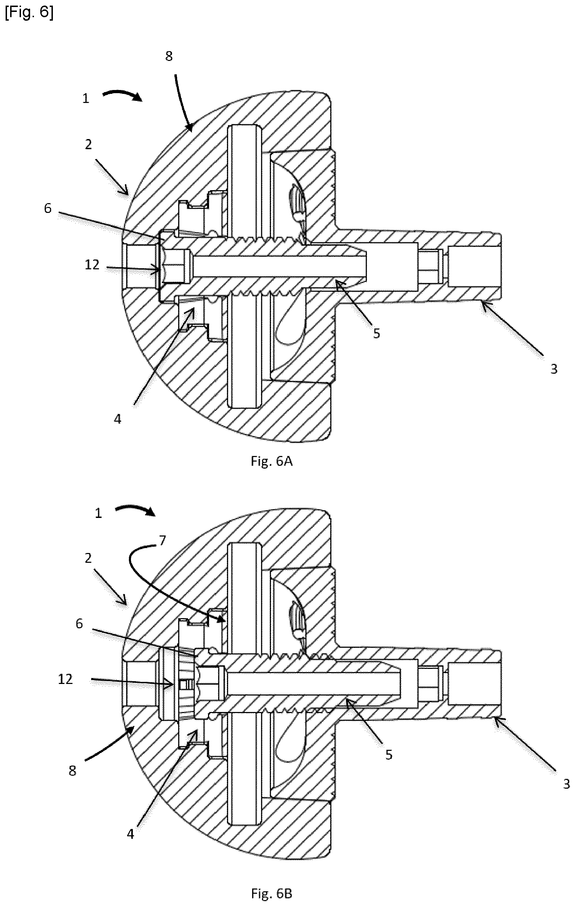

Selon une première variante de ce mode de réalisation de l'invention (figures 1A à 1C), applicable sur l'ensemble des modes de réalisation, la bague rapportée 4 est vissée au sein du logement 9 de la glénosphère 2 qui comprend pour ce faire un taraud 10 de forme complémentaire au filet radial externe 11 de la bague rapportée 4. Lorsque la bague rapportée 4 est en position finale au sein de logement 9 de la glénosphère 2 (c'est-à-dire une fois qu'elle est vissée complètement), elle forme avec le logement une chambre 12 qui emprisonne la tête 6 de la visse de serrage 5, limitant cette dernière dans ses mouvements. La bague rapportée 4 présente une surface d'appui 7 (visible sur la figure 6B) contre laquelle vient buter la tête 6 de la vis de serrage 5 lors du serrage de la glénosphère 2 sur la métaglène 3. Ainsi les efforts issus de ce serrage sont répartis de manière homogène sur l'ensemble de la surface de contact entre le logement 9 de la glénosphère 2 et la bague rapportée 4. Cette large surface qui comprend notamment toute la surface radiale de la bague rapportée permet de minimiser la force des contraintes subies localement par la glénosphère 2 en céramique, ce qui réduit le risque de fissures ou de cassure de cette dernière.

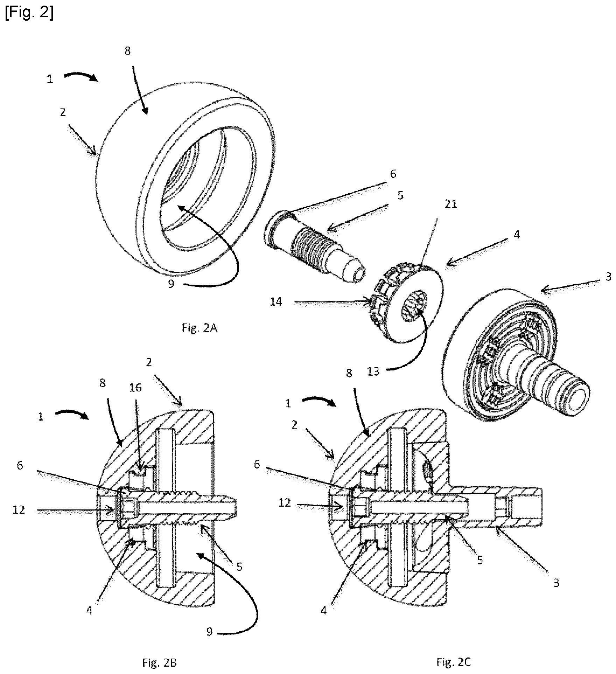

Selon une deuxième variante de ce mode de réalisation de l'invention (figures 2A à 2C), applicable sur l'ensemble des modes de réalisation, la bague rapportée 4 est clipsée au sein du logement 9 de la glénosphère via des moyens de clipsage formant ainsi avec le logement 9 la chambre 12 emprisonnant la tête 6 de la vis de serrage 5. Plus précisément, les moyens de clipsage se matérialisent, dans cette variante, par des dents 14 présentes aux extrémités des ailettes 21, formant ensemble une rainure, et par une languette 16 prévue au sein du logement 9 et dont la forme permet de recevoir la rainure formée par les dents 14 de sorte à permettre le clipsage la bague rapportée 4. Comme pour l'ensemble des modes de réalisation et variantes de l'invention, la bague rapportée 4 comprend une surface d'appui 7 formant butée pour la tête 6 de la vis 5 lors du serrage de cette dernière. La bague rapportée 4 comprend selon cette variante des moyens périphériques 21 élastiquement déformables et configurés pour se déformer au passage de ladite vis de serrage 5 (la position finale en butée de la vis de serrage est représentée sur la figure 6B). Ces moyens périphériques déformables 21 peuvent comprendre des ailettes 21 déformables qui viennent s'expandre sur l'extérieur lorsqu'elles sont contraintes par le passage de la vis de serrage 5, en prenant appui de façon homogène sur la face interne du logement 9 de la glénosphère 2 en céramique. Ainsi et comme mentionné pour la première variante, il en résulte une répartition plus homogène des contraintes issues du serrage de la glénosphère 2 sur la métaglène 3, ce qui participe in fine à une réduction de la force des contraintes subies localement par la glénosphère 2 en céramique, réduisant le risque de fissures ou de cassure de cette dernière.

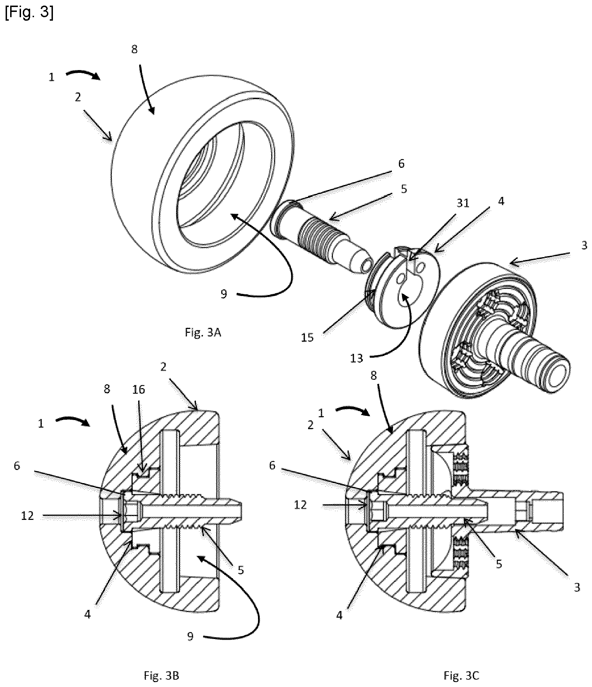

Selon une troisième variante de ce mode de réalisation de l'invention (figures 3A à 3C), applicable sur l'ensemble des modes de réalisation, la bague rapportée 4 est clipsée au sein du logement 9 de la glénosphère 2 via des moyens de clipsage formant ainsi avec le logement 9 la chambre 12 emprisonnant la tête 6 de la vis de serrage 5. Plus précisément, les moyens de clipsage se matérialisent, dans cette variante, par une rainure 15 (présente sur la bague rapportée 4) et une languette 16 (prévue au sein du logement 9) dont la forme permet de recevoir la rainure 15 de sorte à permettre le clipsage la bague rapportée 4. Comme pour l'ensemble des modes de réalisation et variante de l'invention, la bague rapportée 4 comprend une surface d'appui 7 formant butée pour la tête 6 de la vis de serrage 5 lors du serrage de cette dernière. La bague rapportée 4 selon cette variante présente une fente 31. Cette bague 4 fendue présente donc une forme en U qui lui permet de s'expandre sur l'extérieur sous l'impulsion de la vis de serrage 5 (qui présente une portion dont le diamètre est supérieur ou égal au diamètre de l'orifice central 13 de la bague rapportée). Ainsi la bague rapportée peut prendre appui de façon homogène sur la face interne du logement 9 de la glénosphère 2 en céramique, lors du serrage de la vis de serrage 5. Là-aussi, une répartition plus homogène des contraintes issues du serrage de la glénosphère 2 sur la métaglène 3 est rendue possible.

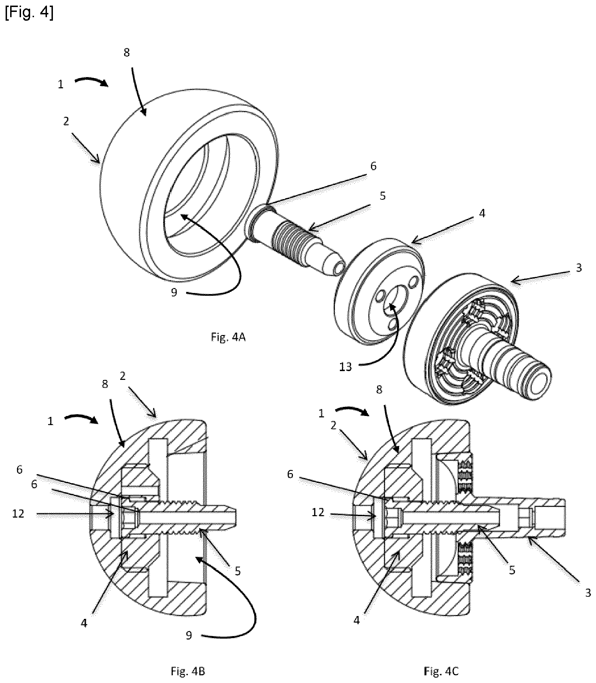

Selon une quatrième variante de ce mode de réalisation de l'invention (figures 4A à 4C), applicable sur l'ensemble des modes de réalisation, la bague rapportée 4 est réalisée dans un matériau thermiquement expansif exerçant une force de pression d'expansion au sein du logement 9 de la glénosphère 2 après avoir été positionné dans ce dernier. Pour ce faire, la bague rapportée 4 peut être refroidie (par exemple dans l'azote) et la glénosphère en céramique peut être chauffée de sorte que l'introduction et le bon positionnement de la bague rapportée 4 au sein du logement 9 de glénosphère est possible. Le diamètre de la bague 4 peut être légèrement plus grand que celui du logement de la glénosphère 2 à température identique, ce qui signifie que lorsque la bague 4 rapportée se réchauffe, elle exerce une force de compression homogène sur l'ensemble de la surface 41 du logement 9 de la glénosphère 2 avec laquelle elle est en contact. A l'instar de l'ensemble des variantes précédemment présentées, une répartition plus homogène des contraintes issues du serrage de la glénosphère 2 sur la métaglène 3 est rendue possible.

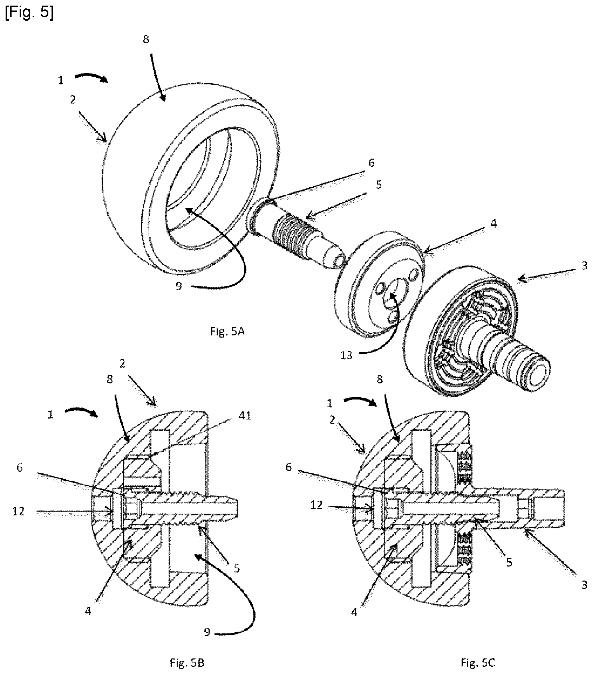

Selon une cinquième variante de ce mode de réalisation de l'invention (figure 5A à 5C), la bague 4 est rapportée brasée avec le logement 9 de la glénosphère 2 (création d'une liaison permanente 41 entre la surface externe de la bague 4 et le logement 9 grâce à la mise en place d'un fil métallique entre ces deux éléments et une mise en température suffisante du fil permettant de créer ladite liaison). Comme pour les précédentes variantes, la bague 4 une fois positionnée au sein du logement 9 de la glénosphère 2 forme avec celui-ci une chambre 12 emprisonnant la tête 6 de la vis de serrage 5. A l'instar des précédentes variante, la bague rapportée 4 selon cette variante comprend également une surface d'appui 7 formant butée pour la tête de la vis de serrage.

Selon une sixième variante de ce mode de réalisation de l'invention (non représentée), la bague rapportée 4 est collée au sein du logement 9 de la glénosphère 2 et présente ainsi les mêmes avantages concernant la répartition des contraintes issues du serrage que ceux mentionnés aux paragraphes précédents.

Dans le second mode de réalisation (figures 7A à 7B), le corps principal 200 correspond à une glène anatomique 200 de prothèse d'épaule dite anatomique. Dans ce mode de réalisation, l'implant 100 comprend au moins deux vis de serrage 5, permettant le serrage directement avec l'os du patient, et au moins deux bagues rapportées 4 (similaires à celle présentée dans le cadre de la variante illustrée aux figures 2A à 2C). Le fonctionnement des deux bagues rapportées 4 est identique à celui présenté pour le premier mode de réalisation (où le corps principal 2 est une glénosphère 2). L'ensemble des variantes présentées dans le cadre du premier mode de réalisation s'appliquent à ce second mode de réalisation, ainsi qu'à tous les modes de réalisation envisageables où l'implant articulaire présente une surface articulaire en céramique et est vissé et serré au moyen d'au moins une vis de serrage, sur l'os du patient ou sur un élément intermédiaire fixé audit os. Chaque bague rapportée 4 forme avec un logement 9 dédié de la glène anatomique 200 une chambre 12 emprisonnant la tête 6 d'une des deux vis de serrage 5. Chaque vis de serrage 5 permettant ici le serrage entre la glène anatomique 200 et l'os du patient (non représenté). Ainsi et comme mentionné pour l'ensemble des variantes du premier mode de réalisation, il en résulte une répartition plus homogène des contraintes issues du serrage de la glène 200 sur l'os du patient, ce qui participe in fine à une réduction de la force des contraintes subies localement par la glène 200 en céramique, réduisant le risque de fissures ou de cassure de cette dernière.

L'invention n'est pas limitée aux modes de réalisation présentés. En effet et comme évoqué précédemment, l'invention peut être appliqué à tout type d'implant articulaire présentant une surface articulaire en céramique et étant vissé et serré au moyen d'au moins une vis de serrage, sur l'os du patient ou sur un élément intermédiaire fixé audit os. A titre d'exemple, l'invention concerne également des implants de prothèses de hanche ou de genou.

### Liste de références

1 : implant selon le premier mode de réalisation
2 : corps principal/glénosphère
3 : métaglène
4 : bague rapportée
5 : vis de serrage
6 : tête de la vis de serrage
7 : surface d'appui
8 : surface externe articulaire
9 : logement du corps principal
10 : taraud
11 : filet
12 : chambre
13 : orifice central de la bague
14 : dent
15 : rainure
16 : languette
21 : ailettes déformables élastiquement
31 : fente
41 : liaison issue du brasage
100 : implant selon le deuxième mode de réalisation
200 : corps principal/glène anatomique

## Revendications

1. Implant de prothèse articulaire (1 ; 100) comprenant :
- un corps principal (2 ; 200) principalement en céramique et comprenant une surface externe articulaire (8) en céramique de forme concave ou convexe,
- au moins une vis de serrage (5) configurée pour permettre le serrage du corps principal (2 ; 200) sur l'os du patient ou sur un élément intermédiaire (3) fixé audit os,
- au moins une bague rapportée (4) dans le corps principal (2 ; 200) et formant avec ce dernier une chambre (12) emprisonnant la tête (6) de la vis de serrage (5) et comprenant une surface d'appui (7) formant butée pour ladite tête (6) de la vis de serrage (5), la bague rapportée (4) étant configurée pour être en contact avec la vis de serrage (5) durant ledit serrage, la bague rapportée (4) comprenant un orifice central (13) traversée par la vis de serrage (5),
**caractérisé en ce que** la bague rapportée (4) est configurée pour répartir les contraintes mécaniques issues du serrage de la vis de serrage (5) de manière homogène au sein du corps principal (2 ; 200).

2. Implant (1 ; 100) selon la revendication précédente, dans lequel la bague rapportée (4) comprend une portion filetée (11) et le corps principal (2 ; 200) comprend une portion taraudée (12) correspondante, la portion filetée (11) et la portion taraudée (12) étant configurées pour permettre le vissage de la bague rapportée (4) avec le corps principal (2 ; 200).

3. Implant (1 ; 100) selon la revendication la revendication 1, dans lequel la bague rapportée (4) comprend des moyens de clipsage (14, 15) au corps principal (2 ; 200).

4. Implant (1 ; 100) selon l'une des revendications précédentes, dans lequel la bague rapportée (4) comprend des moyens périphériques déformables élastiquement (21) configurés pour se déformer au passage de ladite vis de serrage (5).

5. Implant (1 ; 100) selon l'une des revendications précédentes, dans lequel la bague rapportée (4) est fendue de sorte à pouvoir s'élargir au passage de ladite vis de serrage (5).

6. Implant (1 ; 100) selon l'une des revendications précédentes, dans lequel la bague rapportée (4) est réalisée dans un matériau thermiquement expansif exerçant une force de pression d'expansion sur le corps principal (2 ; 200).

7. Implant (1 ; 100) selon l'une des revendications précédentes, dans lequel la bague rapportée (4) est rapportée brasée avec le corps principal (2 ; 200).

8. Implant (1 ; 100) selon l'une des revendications précédentes, dans lequel la bague rapportée (4) est collée sur le corps principal (2 ; 200).

9. Implant (1 ; 100) selon l'une des revendications précédentes, dans lequel le corps principal (2) est une glénosphère (2), la vis de serrage (5) étant configurée pour permettre le serrage entre la glénosphère (2) et une métaglène (3) fixée sur la glène du patient.

10. Implant (1 ; 100) selon la revendication précédente, dans lequel la glénosphère (2) a un diamètre compris entre 30 et 46mm.

11. Implant (1 ; 100) selon l'une des revendications 1 à 8, dans lequel le corps principal (2 ; 200) est un plateau tibial céramique, un condyle de genou, une glène anatomique, un cotyle de hanche ou une cupule céramique.

12. Prothèse articulaire comprenant un implant (1 ; 100) selon l'une quelconque des revendications précédentes.

## Patentansprüche

1. Implantat für Gelenkprothese (1; 100), umfassend:
- einen Hauptkorpus (2; 200), der hauptsächlich aus Keramik ist und eine äußere Gelenkfläche (8) aus Keramik konkaver oder konvexer Form umfasst,
- wenigstens eine Klemmschraube (5), die ausgebildet ist, um das Festklemmen des Hauptkorpus (2; 200) auf dem Knochen des Patienten oder auf einem Zwischenelement,(3) das an dem Knochen befestigt ist, zu gestatten,
- wenigstens einen Ring (4), der in den Hauptkorpus (2; 200) eingebracht ist und mit diesem letzten eine Kammer (12) bildet, die den Kopf (6) der Klemmschraube (5) einsperrt, und eine Abstützfläche (7) umfasst, die einen Anschlag für den Kopf (6) der Klemmschraube (5) bildet, wobei der eingebrachte Ring (4) ausgebildet ist, um während des Festklemmens mit der Klemmschraube (5) in Kontakt zu sein, wobei der eingebrachte Ring (4) eine mittige Öffnung (13) umfasst, die von der Klemmschraube (5) durchquert wird,
**dadurch gekennzeichnet, dass** der eingebrachte Ring (4) ausgebildet ist, um die mechanischen Beanspruchungen, die von dem Festklemmen der Klemmschraube (5) stammen, im Inneren des Hauptkorpus (2; 200) gleichmäßig zu verteilen.

2. Implantat (1; 100) nach dem vorhergehenden Anspruch, wobei der eingebrachte Ring (4) einen Außengewindeteil (11) umfasst und der Hauptkorpus (2; 200) einen entsprechenden Innengewindeteil (12) umfasst, wobei der Außengewindeteil (11) und der Innengewindeteil (12) ausgebildet sind, um das Verschrauben des eingebrachten Rings (4) mit dem Hauptkorpus (2; 200) zu gestatten.

3. Implantat (1; 100) nach dem Anspruch 1, wobei der eingebrachte Ring (4) Mittel zum Einrasten (14, 15) an dem Hauptkorpus (2; 200) umfasst.

4. Implantat (1; 100) nach einem der vorhergehenden Ansprüche, wobei der eingebrachte Ring (4) elastisch verformbare umlaufende Mittel (21) umfasst, die ausgebildet sind, um sich beim Durchtritt der Klemmschraube (5) zu verformen.

5. Implantat (1; 100) nach einem der vorhergehenden Ansprüche, wobei der eingebrachte Ring (4) geschlitzt ist, so dass er sich beim Durchtritt der Klemmschraube (5) aufweiten kann.

6. Implantat (1; 100) nach einem der vorhergehenden Ansprüche, wobei der eingebrachte Ring (4) aus einem thermisch expansiven Material realisiert ist, das eine Expansionsdruckkraft auf den Hauptkorpus (2; 200) ausübt.

7. Implantat (1; 100) nach einem der vorhergehenden Ansprüche, wobei der eingebrachte Ring (4) durch mit dem Hauptkorpus (2; 200) verlötet eingebracht ist.

8. Implantat (1; 100) nach einem der vorhergehenden Ansprüche, wobei der eingebrachte Ring (4) auf den Hauptkorpus (2; 200) geklebt ist.

9. Implantat (1; 100) nach einem der vorhergehenden Ansprüche, wobei der Hauptkorpus (2; 200) eine Glenosphäre (2) ist, wobei die Klemmschraube (5) ausgebildet ist, um das Festklemmen zwischen der Glenosphäre (2) und einer Glenoidbasisplatte (3), die auf der Schulterpfanne des Patienten befestigt ist, zu gestatten.

10. Implantat (1; 100) nach dem vorhergehenden Anspruch, wobei die Glenoidbasisplatte (2) einen Durchmesser zwischen 30 und 46 mm hat.

11. Implantat (1; 100) nach einem der Ansprüche 1 bis 8, wobei der Hauptkorpus (2; 200) ein keramisches Schienbeinplateau, eine Kniekondyle, eine anatomische Schulterpfanne, eine Hüftgelenkpfanne oder eine keramische Cupula ist.

12. Gelenkprothese, umfassend ein Implantat (1; 100) nach einem der vorhergehenden Ansprüche.

## Claims

1. Joint prosthesis implant (1; 100) comprising:
- a main body (2; 200) made mainly of ceramic and comprising a joint outer surface (8) made of ceramic, of concave or convex shape,
- at least one tightening screw (5) configured to tighten the main body (2; 200) on the patient's bone or on an intermediate element (3) attached to said bone,
- at least one added ring (4) in the main body (2; 200) and forming with it a chamber (12) trapping the head (6) of the tightening screw (5) and comprising a bearing surface (7) forming a stop for said head (6) of the tightening screw (5), the added ring (4) being configured to be in contact with the tightening screw (5) during said tightening, the added ring (4) comprising a central hole (13) crossed by the tightening screw (5),
**characterized in that** the added ring (4) is configured to distribute homogeneously in the main body (2; 200) the mechanical stresses caused by tightening the tightening screw (5).

2. Implant (1; 100) according to the preceding claim, wherein the added ring (4) comprises a threaded portion (11) and the main body (2; 200) comprises a corresponding tapped portion (12), the threaded portion (11) and the tapped portion (12) being configured so that the added ring (4) can be screwed into the main body (2; 200).

3. Implant (1; 100) according to claim 1, wherein the added ring (4) comprises means (14, 15) for clipping to the main body (2; 200).

4. Implant (1; 100) according to one of the preceding claims, wherein the added ring (4) comprises elastically deformable peripheral means (21) configured to deform as said tightening screw (5) is inserted.

5. Implant (1; 100) according to one of the preceding claims, wherein the added ring (4) is split so that it can expand as said tightening screw (5) is inserted.

6. Implant (1; 100) according to one of the preceding claims, wherein the added ring (4) is made of a thermally expansive material exerting expansion pressure on the main body (2; 200).

7. Implant (1; 100) according to one of the preceding claims, wherein the added ring (4) is added brazed onto the main body (2; 200).

8. Implant (1; 100) according to one of the preceding claims, wherein the added ring (4) is glued onto the main body (2; 200).

9. Implant (1; 100) according to one of the preceding claims, wherein the main body (2) is a glenosphere (2), the tightening screw (5) being configured to tighten the glenosphere (2) onto a metaglene (3) attached to the patient's glenoid.

10. Implant (1; 100) according to the preceding claim, wherein the diameter of the glenosphere (2) is between 30 and 46 mm.

11. Implant (1; 100) according to one of claims 1 to 8, wherein he main body (2; 200) is a ceramic tibial plateau, a knee condyle, an anatomic glenoid, a hip acetabulum or a ceramic cup.

12. Joint prosthesis comprising an implant (1; 100) according to any one of the preceding claims.
